# FASCICULE DE BREVET EUROPEEN

(11) **EP 1 001 775 B1**
(45) Date de publication et mention de la délivrance du brevet: **06.02.2002**
(21) Numéro de dépôt: 98935102.8
(22) Date de dépôt: 06.07.1998
(51) Int. Cl.: A61K 31/475, A61K 31/195, A61P 15/10

(54) **MEDICAMENT, CONTENANT DE LA YOHIMBINE ET DE L'ARGININE, DESTINE A TRAITER LES DYSFONCTIONS ERECTILES**
YOHIMBINE UND ARGININ ENTHALTENDE ZUSAMMENSETZUNG ZUR BEHANDLUNG DER EREKTILEN FEHLFUNKTION
MEDICINE CONTAINING YOHIMBINE AND ARGININE FOR TREATING ERECTILE DYSFUNCTION

(30) Priorité: 04.07.1997 FR 9708504
(43) Date de publication de la demande: 24.05.2000
(73) Titulaire: Real 2000 Limited, Cork (IE)
(72) Inventeur: GORNY, Philippe, F-75116 Paris (FR)
(74) Mandataire: Tonnellier, Jean-Claude
(86) Numéro de dépôt international: FR9801438
(87) Numéro de publication internationale: WO9901132

(56) Documents cités:
- WO-A-95/05172
- DATABASE EMBASE ELSEVIER SCIENCE PUBLISHERS, AMSTERDAM, NL DELLA MORTE E. ET AL: "Impotence: Non-hormonal pharmacotherapy. Old and new drugs" XP002055796 & ARCHIVIO ITALIANO DI UROLOGIA E ANDROLOGIA, 1995, 67/5 (321-327), ITALY

## Description

L'invention concerne l'utilisation, en association, de yohimbine et d'arginine dans la préparation d'un médicament destiné à traiter les dysfonctions érectiles.

On sait que le processus de l'érection est schématiquement celui indiqué ci-après. Le tissu érectile de la verge, appelé corps caverneux, est un tissu spongieux capable de se remplir de sang. Au repos, les artères de la verge sont sous la dépendance du tonus adrénergique qui les maintient spasmées, de sorte qu'aucun flux sanguin ne vient remplir le corps caverneux. En cas de stimulation appropriée, les nerfs érecteurs inhibent le tonus adrénergique, les artères de la verge se dilatent, et le corps caverneux se remplit de sang, grossit, et l'augmentation de la pression interne fait qu'il devient rigide. En grossissant, il écrase les veines de la verge, empêchant l'évacuation du sang qu'il contient, ce qui assure le maintien de la rigidité. Après l'éjaculation, l'adrénaline est à nouveau libérée localement, l'apport en sang artériel se réduit aussitôt, la pression dans le corps caverneux diminue et le sang accumulé dans celui-ci peut s'évacuer par les veines qui ne sont plus comprimées, ce qui entraîne la perte de la rigidité, avec retour à l'état de repos.

Il est connu qu'une proportion assez importante d'hommes souffre de dysfonctions érectiles permanentes ou temporaires. Ces troubles peuvent être d'origine organique, auquel cas ils nécessitent des traitements spécifiques adaptés à chaque cause. Mais on observe une majorité de dysfonctions érectiles non organiques, souvent d'origine psychogène. Pour ces derniers cas, divers traitements sont disponibles. L'injection intra-caverneuse de substances vaso-actives est susceptible de fournir de bons résultats, mais est difficilement acceptée par une proportion importante de patients. Les traitements par voie orale sont généralement mieux acceptés. On a proposé à cet effet divers produits, souvent d'origine végétale : ginseng, gingembre, yohimbine, noix de kola, spiruline, ylang-ylang, berce, cannelle, etc.

La yohimbine est une substance extraite de l'écorce de la plante Corynanthe yohimbe. Elle a des propriétés d'antagoniste des récepteurs alpha-2-adrénergiques présynaptiques. Autrement dit, elle inhibe les effets de l'adrénaline et favorise ainsi l'irrigation du corps caverneux par le sang artériel. Elle a été proposée dans le traitement des impuissances d'origine psychogène. Certaines études publiées à ce sujet font état d'améliorations dans environ 35 à 45 % des cas avec la yohimbine, mais ces résultats sont controversés. En outre, divers effets secondaires tels que vertiges, anxiété, nervosité, céphalées, insomnies et augmentation de la tension artérielle ont été observés, quoique pour des doses relativement élevées ; voir par exemple The Medical Letter, Edition française, vol.17, n° 2, 5-6 (ML USA n° 938), 1995.

On a également proposé l'administration de L-arginine. L'administration d'arginine se traduirait par un effet sur la relaxation musculaire des artères et du corps caverneux, cette relaxation étant nécessaire à l'obtention de l'érection. L'administration de 2800 mg par jour de L-arginine aurait un effet favorable sur les dysfonctions érectiles dans 40 % des cas environ ; voir A.W. ZORGNIOTTI et E.F. LIZZA, Int. J. Impotence Res., 6, 33-36 (1994).

On a maintenant découvert que, dans ce domaine, l'association de la yohimbine et de l'arginine présente des propriétés intéressantes. Des essais ont en effet montré que l'association d'arginine et de yohimbine permet d'obtenir des résultats favorables dans le traitement des dysfonctions érectiles, principalement non organiques, en utilisant des doses de yohimbine plus faibles que celles utilisées antérieurement, grâce à un effet de synergie.

Avec l'association de yohimbine et d'arginine, il est également possible de réduire les doses d'arginine utilisées.

Dans nombre de cas, cette association semble agir assez rapidement et peut donc être utilisée dans le traitement des dysfonctions érectiles temporaires.

L'invention a donc pour objet l'utilisation en association présentant un effet de synergie de yohimbine et d'arginine comme ingrédients actifs dans la préparation d'un médicament destiné à traiter les dysfonctions érectiles non organiques.

La yohimbine et l'arginine peuvent être utilisées sous forme de base libre ou sous forme de sel acceptable en pharmacie.

Les ingrédients actifs, dans le médicament de l'invention, peuvent être présentés de façon séparée, chacun sous une forme pharmaceutique appropriée, et réunis dans un même emballage.

Mais pour faciliter l'administration simultanée des ingrédients actifs, on préfère généralement préparer le médicament de l'invention sous une forme pharmaceutique contenant à la fois la yohimbine et l'arginine.

Le médicament de l'invention peut être préparé sous une forme pharmaceutique permettant l'administration d'une dose suffisante de yohimbine, notamment une dose de 2 à 8 mg, et en particulier de 2 à 6 mg par jour, en une ou deux prises. Cette dose est calculée en poids de yohimbine sous forme de base libre.

Le médicament de l'invention est préparé sous une forme pharmaceutique permettant l'administration d'une dose suffisante d'arginine qui est par exemple une dose de 1 à 4 g par jour, et en particulier de 1 à 2 g par jour, en une ou deux prises, ladite dose étant calculée en poids d'arginine sous forme de base libre.

Le médicament de l'invention peut être administré par voie orale, sublinguale, nasale, rectale ou cutanée.

A cet effet, il peut être présenté sous toute forme permettant l'administration par voie orale (en particulier sous la forme de gélules, de solutions buvables, de granulés, de tablettes ou de comprimés), par voie nasale (solutions à administrer sous forme de gouttes ou de pulvérisations), par voie rectale (suppositoires) ou par voie cutanée (onguents ou patches).

Ces formes pharmaceutiques sont préparées de façon usuelle et peuvent contenir des excipients et véhicules classiques appropriés.

La yohimbine peut être utilisée sous forme de base libre ou sous forme d'un sel tel que le chlorhydrate.

L'arginine peut être utilisée sous la forme de base libre ou d'un sel acceptable en pharmacie, tel que le chlorhydrate, le glutamate, l'aspartate ou le citrate.

La durée du traitement peut varier par exemple de 2 à 4 semaines ou davantage. On peut également envisager une utilisation épisodique.

Les exemples suivants illustrent l'invention.

### EXEMPLE 1

On a préparé une gélule constituée par une capsule de gélatine contenant :
- Arginine : 0,5 g
- Yohimbine : 1 mg

### EXEMPLE 2

On a procédé à des essais randomisés en double aveugle portant sur 42 hommes adultes mariés, âgés de 28 à 64 ans, souffrant de dysfonctions érectiles sans cause organique décelable.

Le traitement consistait à administrer soit (21 cas) 4 gélules telles que décrites à l'exemple 1 ci-dessus (2 le matin et 2 le soir), soit (21 cas), de façon analogue, un placebo (4 gélules d'aspect identique mais contenant uniquement 0,5 g de lactose).

Les personnes sur lesquelles était effectué le test, de même que les personnes fournissant les composés étudiés, ignoraient si elles recevaient ou fournissaient l'association yohimbine + arginine ou le placebo.

Après 2 semaines, on a interrogé les personnes soumises au test en leur demandant si elles ont constaté une amélioration de la fonction érectile.

L'étude des résultats a permis de tirer les conclusions suivantes :
- chez les sujets atteints de dysfonctions érectiles passagères (22 cas), 12 avaient reçu le placebo. On a noté 7 cas d'amélioration avec l'association étudiée et 4 cas d'amélioration avec le placebo ;
- chez les sujets atteints de dysfonctions érectiles chroniques (20 cas), 9 avaient reçu le placebo. On a noté 4 cas d'amélioration avec l'association testée et 1 cas d'amélioration avec le placebo.

## Revendications

1. Utilisation, en association présentant un effet de synergie, de yohimbine et d'arginine comme ingrédients actifs dans la préparation d'un médicament destiné à traiter les dysfonctions érectiles.

2. Utilisation selon la revendication 1, dans laquelle la yohimbine et/ou l'arginine sont utilisées sous forme de base libre ou sous forme de sel.

3. Utilisation selon la revendication 1, dans laquelle on prépare le médicament sous une forme pharmaceutique permettant l'administration simultanée des ingrédients actifs.

4. Utilisation selon l'une quelconque des revendications précédentes, **caractérisée par le fait que** l'on prépare ledit médicament sous une forme pharmaceutique permettant l'administration d'une dose de 2 à 8 mg de yohimbine, en une ou deux prises, ladite dose étant calculée en poids de yohimbine sous forme de base libre.

5. Utilisation selon l'une quelconque des revendications précédentes, **caractérisée par le fait que** l'on prépare ledit médicament sous une forme pharmaceutique permettant l'administration d'une dose de 1 à 4 g, et en particulier de 1 à 2 g d'arginine, en une ou deux prises, ladite dose étant calculée en poids d'arginine sous forme de base libre.

6. Médicament destiné à traiter les dysfonctions érectiles, **caractérisé par le fait qu'**il contient comme ingrédients actifs, en association présentant un effet de synergie, de l'arginine, sous forme libre ou salifiée, et de la yohimbine, sous forme libre ou salifiée.

7. Médicament selon la revendication précédente, **caractérisé par le fait qu'**il contient lesdits ingrédients actifs de façon séparée, dans un même emballage.

8. Médicament selon la revendication 6, **caractérisé par le fait qu'**il se présente sous une forme pharmaceutique unique contenant les deux ingrédients actifs.

9. Médicament selon l'une quelconque des revendications 6 à 8, **caractérisé par le fait qu'**il est présenté sous une forme pharmaceutique permettant l'administration d'une dose de 2 à 8 mg, et en particulier de 2 à 6 mg de yohimbine, en une ou deux prises, ladite dose étant calculée en poids de yohimbine sous forme de base libre.

10. Médicament selon l'une quelconque des revendications 6 à 9, **caractérisé par le fait qu'**il est présenté sous une forme pharmaceutique permettant l'administration d'une dose de 1 à 4 g, et en particulier de 1 à 2 g d'arginine, en une ou deux prises, ladite dose étant calculée en poids d'arginine sous forme de base libre.

11. Médicament selon l'une quelconque des revendications 6 à 10, **caractérisé par le fait qu'**il se présente sous la forme de gélules, de solutions buvables, de granulés, de tablettes, de comprimés, d'onguents, de patches, de suppositoires ou de solutions nasales à administrer sous forme de gouttes ou pulvérisations.

## Claims

1. Use, in combination exhibiting a synergistic effect, of yohimbine and arginine as active ingredients in the preparation of a medicinal product intended for treating erectile dysfunction.

2. Use according to Claim 1, in which the yohimbine and/or arginine are used in the form of the free base or in the form of a salt.

3. Use according to Claim 1, in which the medicinal product is prepared in a pharmaceutical form which allows simultaneous administration of the active ingredients.

4. Use according to any one of the preceding claims, **characterized in that** the said medicinal product is prepared in a pharmaceutical form which allows the administration of a dose of from 2 to 8 mg of yohimbine, taken in one or two doses, the said dose being calculated as the weight of yohimbine in the form of the free base.

5. Use according to any one of the preceding claims, **characterized in that** the said medicinal product is prepared in a pharmaceutical form which allows the administration of a dose of from 1 to 4 g, and in particular from 1 to 2 g, of arginine, taken in one or two doses, the said dose being calculated as the weight of arginine in the form of the free base.

6. Medicinal product intended for treating erectile dysfunction, **characterized in that** it contains as active ingredients, in combination exhibiting a synergistic effect, arginine, in free or salified form, and yohimbine, in free or salified form.

7. Medicinal product according to the preceding claim, **characterized in that** it contains the said active ingredients separately, in the same packaging.

8. Medicinal product according to Claim 6, **characterized in that** it is in a single pharmaceutical form containing the two active ingredients.

9. Medicinal product according to any one of Claims 6 to 8, **characterized in that** it is in a pharmaceutical form which allows the administration of a dose of from 2 to 8 mg, and in particular from 2 to 6 mg, of yohimbine, taken in one or two doses, the said dose being calculated as the weight of yohimbine in the form of the free base.

10. Medicinal product according to any one of Claims 6 to 9, **characterized in that** it is in a pharmaceutical form which allows the administration of a dose of from 1 to 4 g, and in particular from 1 to 2 g, of arginine, taken in one or two doses, the said dose being calculated as the weight of arginine in the form of the free base.

11. Medicinal product according to any one of Claims 6 to 10, **characterized in that** it is in the form of gel capsules, drinkable solutions, granules, lozenges, tablets, ointments, patches, suppositories or nasal solutions to be administered in the form of drops or sprays.

## Patentansprüche

1. Gemeinsame Verwendung mit synergistischem Effekt von Yohimbin und Arginin als aktive Bestandteile bei der Herstellung eines Medikaments, bestimmt zur Behandlung von erektilen Dysfunktionen.

2. Verwendung gemäß Anspruch 1, worin das Yohimbin und/oder das Arginin in Form der freien Base oder in Form des Salzes verwendet werden.

3. Verwendung gemäß Anspruch 1, worin man das Medikament in einer pharmazeutischen Form herstellt, die die gleichzeitige Verabreichung der Bestandteile gestattet.

4. Verwendung gemäß einem der vorstehenden Ansprüche, **dadurch gekennzeichnet, dass** man das Medikament in einer pharmazeutischen Form herstellt, welche die Verabreichung einer Dosis von 2 bis 8 mg Yohimbin in einer oder zwei Chargen gestattet, wobei die Dosis in Gewicht des Yohimbins in Form der freien Base berechnet ist.

5. Verwendung gemäß einem der vorstehenden Ansprüche, **dadurch gekennzeichnet, dass** man das Medikament in einer pharmazeutischen Form herstellt, welche die Verabreichung einer Dosis von 1 bis 4 g und insbesondere von 1 bis 2 g Arginin in einer oder zwei Chargen gestattet, wobei die Dosis in Gewicht des Arginins in Form der freien Base berechnet ist.

6. Medikament, bestimmt zur Behandlung von erektilen Dysfunktionen, **dadurch gekennzeichnet, dass** es als aktive Bestandteile, die gemeinsam einen synergistischen Effekt aufweisen, Arginin in freier oder versalzter Form und Yohimbin in freier oder versalzter Form enthält.

7. Medikament gemäß dem vorstehenden Anspruch, **dadurch gekennzeichnet, dass** es die besagten aktiven Bestandteile in getrennter Form in derselben Verpackung enthält.

8. Medikament gemäß Anspruch 6, **dadurch gekennzeichnet, dass** es in Form eines einzigen Pharmazeutikums vorliegt, enthaltend beide aktiven Bestandteile.

9. Medikament gemäß einem der Ansprüche 6 bis 8, **dadurch gekennzeichnet, dass** es in einer pharmazeutischen Form vorliegt, gestattend die Verabreichung einer Dosis von 2 bis 8 mg und insbesondere 2 bis 6 mg Yohimbin in einer oder zwei Chargen, wobei die Dosis in Gewicht des Yohimbins in Form der freien Base berechnet ist.

10. Medikament gemäß einem der Ansprüche 6 bis 9, **dadurch gekennzeichnet, dass** es in einer pharmazeutischen Form vorliegt, gestattend die Verabreichung einer Dosis von 1 bis 4 g und insbesondere 1 bis 2 g Arginin in einer oder zwei Chargen, wobei die Dosis in Gewicht des Arginins in Form der freien Base berechnet ist.

11. Medikament gemäß einem der Ansprüche 6 bis 10, **dadurch gekennzeichnet, dass** es in der Form von Dragees, Trinklösungen, Granulaten, Tabletten, Pillen, Salben, Lappen bzw. Pflastern, Suppositorien bzw. Zäpfchen oder nasalen Lösungen zum Verabreichen in Form von Tröpfchen oder über Zerstäubung vorliegt.
